# EUROPEAN PATENT APPLICATION

(11) **EP 2 460 497 A1**
(43) Date of publication of application: **06.06.2012**
(21) Application number: 09847800.1
(22) Date of filing: 28.07.2009
(51) Int. Cl.: A61F 13/15, A61F 13/472

(54) **DISPOSABLE COMPOSITE PAD FOR TREATING BODY FLUID**

(71) Applicant: Uni-charm Corporation, Ehime 799-0111 (JP)
(72) Inventor: SUGA, Ayami, Kanonji-shi Kagawa 769-1602 (JP); KOSAKO, Yusuke, Kanonji-shi Kagawa 769-1602 (JP)
(74) Representative: Vaughan, Jennifer Ann
(86) International application number: PCT/JP2009/063444
(87) International publication number: WO 2011/013208

(57) **Abstract**

The present invention aims to provide a disposable bodily fluid absorbent composite pad improved so that, when it is desired to peel off each of component pads from the composite pad, an appropriate position of the composite pad to be pinched by the finger can be easily recognized. A menstruation napkin 1 as a typical example of the disposable bodily fluid absorbent composite pad includes two or more component pads layered one on another. These component pads are temporarily joined together along temporarily joined regions. A first component pad 10 is formed with substantially continuous temporarily joined region 13 which is interrupted by a non-joined region on the upper portion of the accompanying drawings. In the non-joined region 14, a graphic of heart mark is displayed as an indicator element 61 so as to be visually recognized through a liquid-absorbent sheet 11.

## Description

### TECHNICAL FIELD

The present invention generally relates to disposable bodily fluid absorbent composite pads including two or more component pads layered one on another and, more particularly, to such composite pads used, for example, as menstruation napkins, urine absorbent pads or panty liners.

### RELATED ART

Disposable bodily fluid absorbent composite pads including two or more component pads layered one on another are known. For example, PATENT CITATION 1 discloses such a composite pad including two or more layered liquid-absorbent component pads temporarily joined together along transversely opposite side edges by locally crimping these layered component pads. Each component pad may be peeled off one by one from a skin-facing side of the composite pad. More specifically, when the component pad contacting the wearer's skin is soiled, it may be peeled off from the remaining components to expose another fresh component pad.

### PRIOR ART DOCUMENT

### PATENT CITATION

PATENT CITATION 1: JP 4226086 B2

### SUMMARY OF THE INVENTION

### PROBLEM TO BE SOLVED BY THE INVENTION

The composite pad of the prior art is designed so that the soiled component pad is peeled off from the composite pad by pinching the end portion thereof with the fingers and pulling it off from the composite pad. To ensure the soiled component pad is smoothly peeled off from the composite pad, it is required that the wearer pinches non-crimped region of the composite pad, because it will be difficult to peel off the soiled component pad starting from the crimped region. However, the composite pad is usually of the same color overall and it is difficult for the wearer to catch sight of the non-crimped region instantly.

An object of the present invention is to provide a disposable bodily fluid absorbent composite pad improved so that the appropriate region to be pinched with the fingers can be easily recognized when it is desired to peel off the soiled component pad from the composite pad.

### MEANS TO SOLVE THE PROBLEM

The present invention relates to an improvement of a disposable bodily fluid absorbent composite pad having a longitudinal direction and a transverse direction, and including a skin-facing side, a non-skin-facing side opposite to the skin-facing side and two or more component pads each including an upper liquid-absorbent sheet on the skin-facing side and a lower liquid-impervious sheet on the non-skin-facing side and layered one on another.

The disposable bodily fluid absorbent composite pad is **characterized in that**:
the two or more component pads respectively include temporarily joined regions along which the component pads are temporarily joined to each other so as to be peeled off from other component pads and non-joined regions; and
each of the component pads is formed with an indicator element adapted to be visually recognized through the liquid-absorbent sheet and the indicator elements are formed in the non-joined regions defined along peripheral edges of the component pads and adapted to indicate at least partially the non-joined regions.

According to one embodiment of the present invention, the temporarily joined regions are formed substantially in continuous fashion along peripheral edges of the component pads and the non-joined regions are defined along partial segments of the peripheral edges so as to interrupt the substantially continuous temporarily joined regions.

According to another embodiment of the present invention, the indicator element includes at least any one of a letter, a symbol and a graphic displayed in the non-joined regions.

According to yet another embodiment of the present invention, the indicator element includes at least any one of a letter, a symbol and a graphic serving to indicate the non-joined regions.

According to still another embodiment of the present invention, the indicator element is defined by coloring of the non-joined regions.

According to yet another embodiment of the present invention, the indicator element includes cutouts formed by partially cutting away the component pads.

### Effect of the invention

Two or more liquid-absorbent component pads layered one on another respectively have temporarily joined regions along which these component pads are detachably and temporarily joined together and non-joined regions. The respective component pads are formed with indicator elements adapted to be visually recognized through the associated liquid-absorbent sheets and to indicate the non-joined regions. In this way, the appropriate position of the composite pad to be pinched with the fingers can be easily recognized as instructed by the indicator element.

### BRIEF DESCTIPTION OF THE DRAWINGS

[FIG. 1] Fig. 1 is a perspective view of a menstruation napkin.
[FIG. 2] Fig. 2 is a sectional view taken along line II-II in Fig. 1.
[FIG. 3] Fig. 3 is a schematic diagram illustrating Example 1.
[FIG. 4] Fig. 4 is a schematic diagram illustrating Example 2.
[FIG. 5] Fig. 5 is a schematic diagram illustrating Example 3.
[FIG. 6] Fig. 6 is a schematic diagram illustrating Example 4.
[FIG. 7] Fig. 7 is a schematic diagram illustrating Example 5.
[FIG. 8] Fig. 8 is a schematic diagram illustrating Example 6.
[FIG. 9] Fig. 9 is a schematic diagram illustrating Example 7.

### IDENTIFICATION OF REFERENCE NUMERALS USED IN THE DRAWINGS

- 1: menstruation napkin (disposable bodily fluid absorbent composite pad)
- 10: first pad (pad)
- 11: liquid-absorbent sheet
- 12: liquid-impervious sheet
- 13: temporarily joined region
- 14: non-joined region
- 20: second pad (pad)
- 21: liquid-absorbent sheet
- 22: liquid-impervious sheet
- 23: temporarily joined region
- 24: non-joined region
- 30: third pad (pad)
- 31: liquid-absorbent sheet
- 32: liquid-impervious sheet
- 33: temporarily joined region
- 34: non-joined region
- 61-63: indicator elements
- 65-67: indicator elements
- 68: cutout
- 69: colored region
- 70: cutout
- 71: colored region
- X: transverse direction
- Y: longitudinal direction

### DETAILED DESCRIPTION OF THE PREFERRED EMBODIMENTS

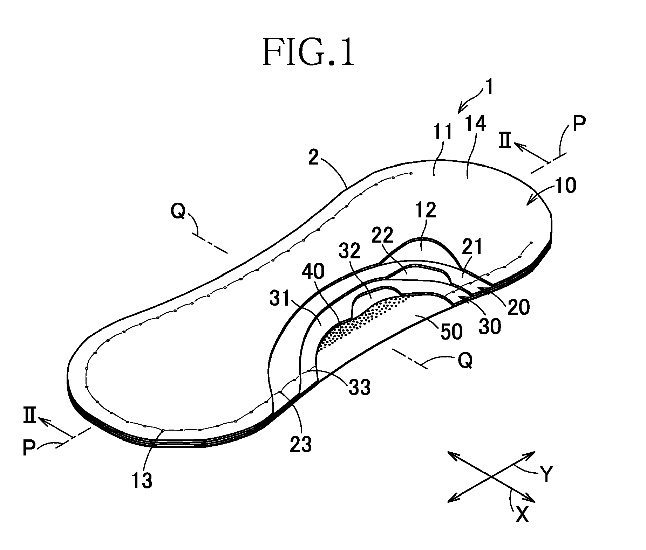

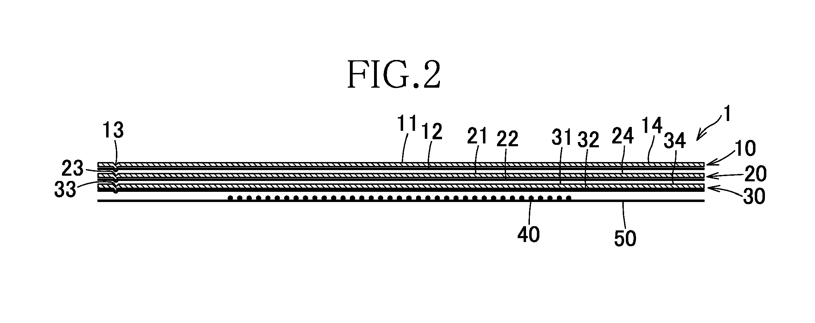

Details of the disposable bodily fluid absorbent composite pad according to the present invention will be described hereunder on the basis of a menstruation napkin as one typical embodiment of the invention. The construction of the menstruation napkin 1 shown in Figs. 1 and 2 is common to Examples 1 through 7. Fig. 1 is a perspective view showing the menstruation napkin 1 as partially cutaway for convenience of illustration and Fig. 2 is a sectional view taken along line II-II in Fig. 1. The menstruation napkin 1 has a longitudinal direction Y and a transverse direction X wherein the menstruation napkin 1 is relatively long in the longitudinal direction Y and has a width gradually reduced from longitudinally opposite ends toward a central region. The menstruation napkin 1 has the imaginary longitudinal center line P-P bisecting a width dimension in the transverse direction X and the imaginary transverse center line Q-Q bisecting a length dimension in the longitudinal direction Y. The menstruation napkin 1 is shaped substantially symmetrically about the imaginary longitudinal center line P-P as well as about the imaginary transverse center line Q-Q.

The menstruation napkin 1 has a skin-facing side facing the wearer's body and a non-skin-facing side facing the wearer's garment. The menstruation napkin 1 includes two or more liquid-absorbent component pads layered from the skin-facing side facing the wearer's body to the non-skin-facing side facing the wearer's garment. Specifically, the menstruation napkin 1 includes a first component pad 10 defining the uppermost layer of the laminate, a second component pad 20 underlying the first component pad 10 and a third component pad 30 underlying the second component pad 20. These first, second and third component pads 10, 20, 30respectively include upper liquid-absorbent sheets 11, 21, 31 and lower liquid-impervious sheets 12, 22, 32. The liquid-absorbent sheets 11, 21, 31 may be formed of a spun lace fibrous non-woven fabric of hydrophilic fibers such as cotton having a basis mass in a range of about 15 to about 40g/m², preferably in a range of about 20 to about 35g/m², more preferably of about 30g/m². The liquid-impervious sheets 12, 22, 32 may be formed of a plastic film having a basis mass in a range of about 10 to about 25g/m², preferably of about 18g/m². The liquid-impervious sheets 12, 22, 32 preferably have a breathability.

The non-skin-facing side, i.e., the side facing the wearer's garment, of the third component pad 30 is formed with joining means 40 such as adhesive which is, in turn, covered from below with a releasable sheet 50. The releasable sheet 50 may be peeled off from the third pad 30 to expose the joining means 40 by the intermediary of which the third pad 30 may be fastened to the wearer's undergarment.

The first, second and third component pads 10, 20, 30 and the releasable sheet 50 are the same in shape as well as in size to one another and respectively have a dimension in the transverse direction X gradually reduced from longitudinally opposite ends toward a central region defined by the imaginary transverse center line Q-Q. By dimensioning the component pads 10, 20, 30 in this manner, it is ensured that these component pads are put in close contact with the wearer's body without irregular deformation of these component pads in the crotch region.

The first, second and third component pads 10, 20, 30 are formed along the peripheral edge 2 thereof with individual temporarily joined regions 13, 23, 33. Specifically, these individual temporarily joined regions 13, 23, 33 include a plurality of depressions (debossings) arranged intermittently along the peripheral edge 2. The depressions are formed by collectively hot-pressing these first, second and third component pads 10, 20, 30 in the thickness direction so as to define the continuous linear temporarily joined region along which these component pads may be temporarily joined together. Each of the temporarily joined regions is formed continuously so that the respective depressions are contiguous one to another and define the linear temporarily joined region or so that the respective depressions are arranged intermittently and collectively define the substantially continuous linear temporarily joined region. The temporary joints between the temporarily joined regions 13, 23, 33 are easily peeled off by pulling upward the respective component pads 10, 20, 30 upwards, so as to be spaced from one another.

Each of these first, second and third component pads 10, 20, 30 has a non-joined region 14, 24 or 34 along a part of its peripheral edge 2 where the temporarily joined region is not formed. In other words, these non-joined regions 14, 24, 34 are formed so as to partially interrupt the continuous temporarily joined regions 13, 23, 33, respectively. It should be appreciated that the position at which the non-joined region 14, 24 or 34 is formed is not limited to that in the embodiment shown by way of example in Fig. 1 but may be formed in other various patterns. The respective component pads 10, 20, 30 may be formed with indicator elements serving to indicate the positions of these non-joined regions 14, 24, 34.

### EXAMPLE 1

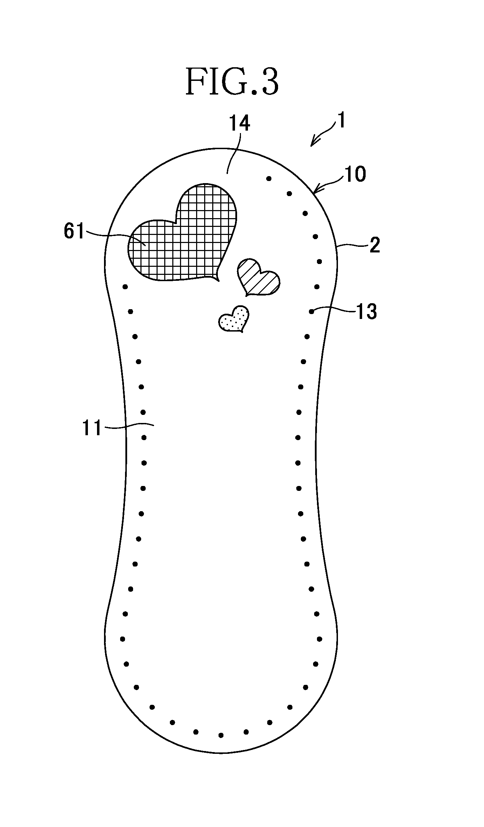

Fig. 3 shows Example 1 in which graphics are used as an indicator element 61. The indicator element 61 seen in Fig. 3 is visually recognized on the first component pad 10. The first component pad 10 is formed with the substantially continuous temporarily joined region 13 which is interrupted by the non-joined region 14 as shown in the upper area of Fig.3 as viewed in the longitudinal direction Y. Heart marks as the indicator element 61 are provided in the non-joined region 14 so as to be visually recognized through the liquid-absorbent sheet 11. These heart marks facilitate the wearer to recognize the non-joined region 14 and to pinch the peripheral edge 2 in the vicinity of these heart marks with the fingers and thereby to peel off the first component pad 10.

While the first component pad 10 alone is illustrated in this Example 1, it will be understood that the second component pad 20 also is formed in the non-joined region 24 with similar graphics. Thereby, the position of the second component pad 20 to be pinched by the wearer can also be easily recognized. While it is unnecessary to peel off the third component pad 30, the third component pad 30 also may be optionally provided with similar heart marks. In this case, the number of the heart marks may be varied in the order of the first, second and third component pads 10, 20, 30 to make the wearer recognize what number component pad is being used. It should be appreciated that the graphics are not limited to the heart marks and may be selected from various graphics such as snow crystal marks, flower marks, and the like.

### EXAMPLE 2

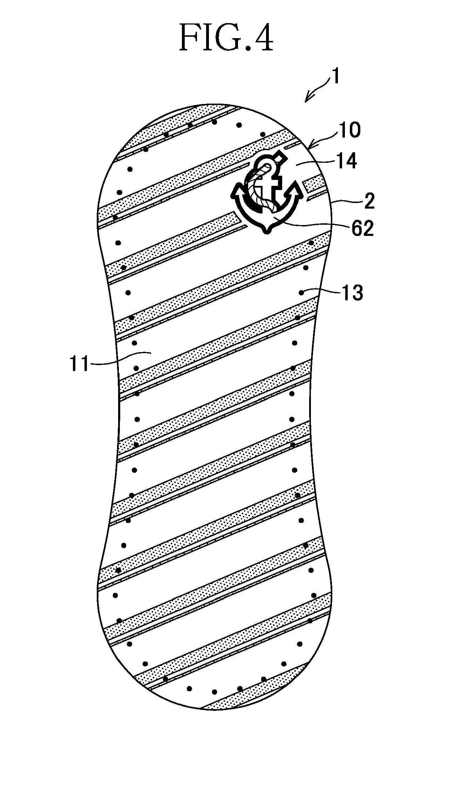

Fig. 4 is a view similar to Fig. 3, showing Example 2 wherein a graphic is used as an indicator element 62. The first component pad 10 is formed along the peripheral edge 2 with the temporarily joined regions 13 defining the substantially continuous temporarily joined line which is interrupted by non-joined region 14 as shown in the upper area of Fig. 4. In the non-joined region 14, an anchor mark is provided as the indicator element 62. In this Example, in addition to the anchor mark as the indicator element 62, another pattern is formed so that both the anchor mark and the pattern may be seen through the liquid-absorbent sheet 11. Specifically, the first component pad 10 is formed overall with a striped pattern. Also when the first component pad 10 is formed with the striped pattern, the non-joined region 14 can be easily recognized by forming the first component pad 10 with a graphic distinguished from such striped pattern.

Similarly to Example 1, the second component pad 20 and the third component pad 30 also may be provided with the same indicator element 62 and pattern as those provided on the first component pad 10. In this case, the pattern and the color thereof on the second component pad 20 and the third component pad 30 may be differentiated form those on the first component pad 10 to ensure that the wearer can identify the respective component pads from one another.

### EXAMPLE 3

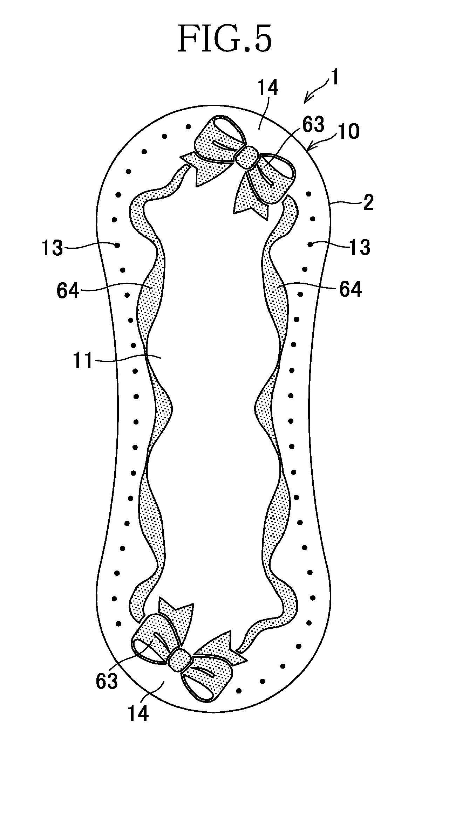

Fig. 5 is a view similar to Fig. 3, showing indicator elements 63 used in Example 3. The first component pad 10 is formed along transversely opposite side edges with two temporarily joined regions 13, respectively, and two non-joined regions 14 are defined between these two temporarily joined regions 13, as shown in the upper and lower areas of Fig. 5. In the respective non-joined regions 14, graphics of knotted ribbon are displayed as the indicator elements 63 and, along the respective temporarily joined regions 13, graphics 64 of ribbon pieces without knots are displayed.

The presence of graphics 64 displayed along the respective temporarily joined regions 13 assists the wearer to recognize the presence of these temporarily joined regions 13. The indicator elements 63 cooperate with the graphics 64 to clarify differences between the non-joined regions 14 and the temporarily joined regions 13 so that the wearer can recognize the non-joined regions 14 more easily.

It should be appreciated that it is not essential for the respective graphics 64 to fall precisely on the associated temporarily joined regions 13 and the respective graphics 64 may be sufficiently nearby the associated temporarily joined regions 13 to distinguish the temporarily joined regions 13 from the non-joined regions 14. It is also possible to use flower pieces as the indicator elements 63, for example, and to use graphics of ivy leaves as the graphics 64 so that the indicator elements 63 and the graphics 64 may be meaningfully associated one with another. The indicator elements 63 and the graphics 64 used for the first component pad 10 may be used for the second component pad 20 and the third component pad 30 also.

### EXAMPLE 4

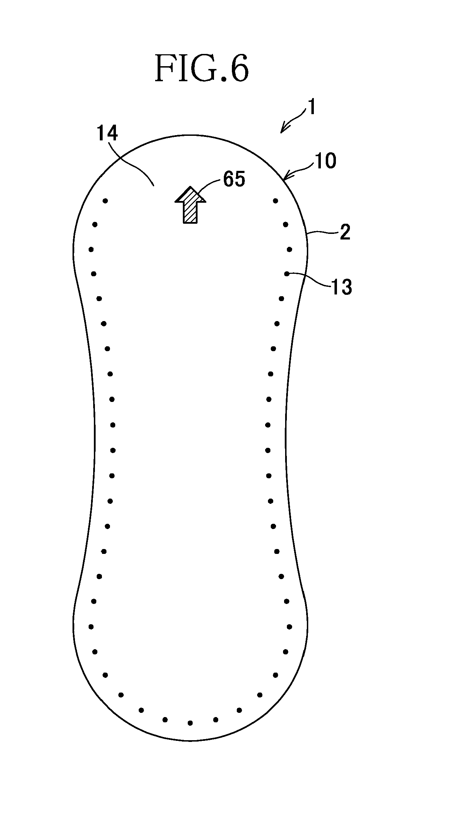

Fig. 6 illustrates Example 4 wherein a symbol is used as an indicator element 65. The first component pad 10 is formed along the peripheral edge 2 with the substantially continuous temporarily joined region 13 which is interrupted by the non-joined region 14 as shown in the upper area of Fig. 6 as viewed in the longitudinal direction Y. In the vicinity of the non-joined region 14, an arrowhead symbol is displayed as the indicator element 65 serving to indicate the presence of the non-joined region 14.

The indicator element 65 is not limited to the arrowhead symbol and various symbols other than the arrowhead symbol may be used as the indicator element 65 so far as the non-joined region can be thereby indicated. For example, the non-joined region 14 can be indicated with graphics of fingers or triangle. As in the case of Example 1, the same indicator element 62 and pattern or graphic as those provided on the first component pad 10 may be provided on the second component pad 20 and the third component pad 30 also.

### EXAMPLE 5

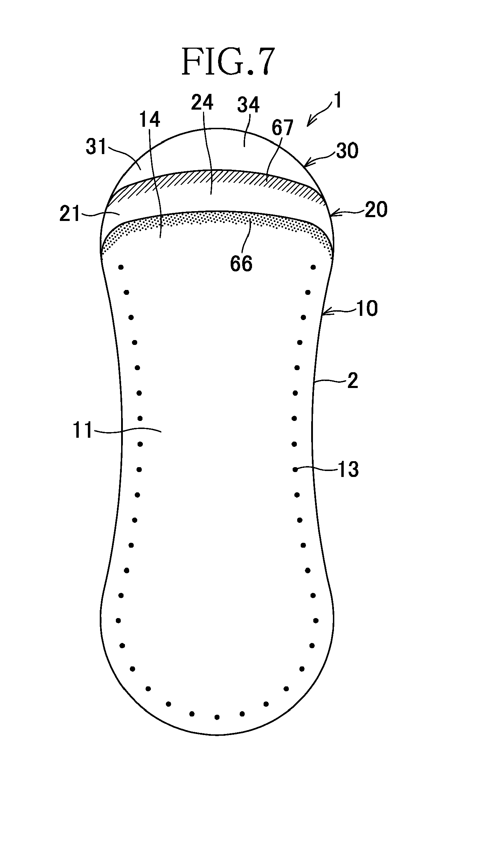

Fig. 7 illustrates Example 5 wherein the indicator elements are colored so as to be distinguished from other regions. The first, second and third component pads 10, 20, 30 are formed along the peripheral edge 2 with the substantially continuous temporarily joined regions 13, 23, 33 which are, in turn, interrupted by the non-joined regions 14, 24, 34, respectively, as shown in the upper area of Fig. 7. As shown in the upper area of Fig. 7, one of the opposite ends of the second pad 20 recedes from the corresponding end of the third component pad 30 so that the second component pad 20 has an area smaller than that of the third component pad 30. In other words, the non-joined region 34 of the third component pad 30 extends outward from underneath and beyond the non-joined region 24 of the second component pad 20. Such non-joined region 24 may be colored differently from the other regions to define an indicator element 67.

As shown in the upper area of Fig. 7, one of the opposite ends of first pad 10 recedes from the corresponding end of the second component pad 20 so that the first component pad 10 has an area smaller than that of the second component pad 20. In other words, the non-joined region 24 of the second component pad 20 extends outward from underneath and beyond the non-joined region 14 of the first component pad 10. Such non-joined region 14 is colored differently from the other regions so as to define an indicator element 66. More specifically, this indicator element 66 is colored differently from the indicator element 67 of the second component pad 20.

In the menstruation napkin 1 as has been heretofore described, the non-joined region 24 of the second component pad 20 extends outward from the non-joined region 14 of the first component pad 10 and the non-joined region 34 of the third component pad 30 extends outward from the non-joined region 24 of the second component pad 20. Such arrangement helps the wearer to understand the manner in which these three component pads are layered one on another and to localize the non-joined regions.

### EXAMPLE 6

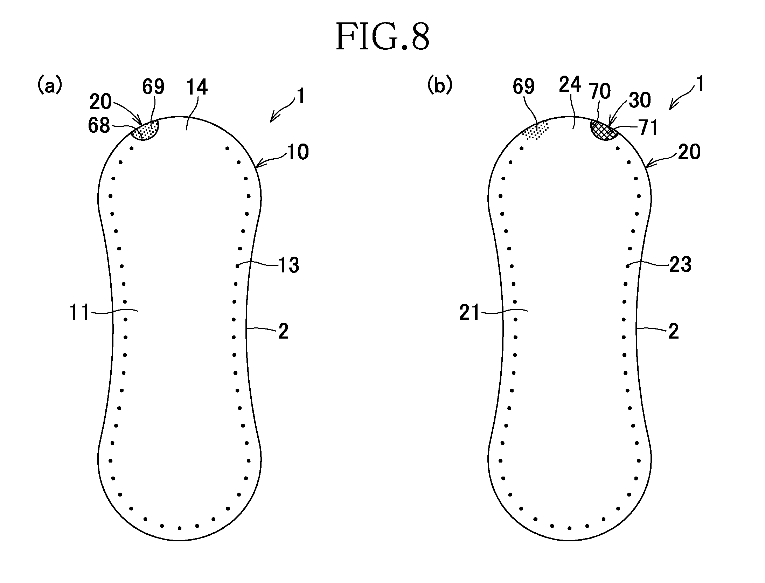

Fig. 8 illustrates Example 6 wherein the pad is formed with cutouts as indicator elements. Specifically, Fig. 8(a) illustrates the state of the composite pad 1 in which the first, second and third component pads 10, 20, 30 are layered one on another and Fig. 8(b) illustrates the state of the composite pad 1 after the first component pad 10 has been peeled off from the state as illustrated in Fig. 8 (a) . The first, second and third component pads 10, 20, 30 are respectively formed along the peripheral edge 2 with the substantially continuous temporarily joined regions 13, 23, 33 which are respectively interrupted by the non-joined regions 14, 24, 34 formed in the upper regions of these component pads, respectively, as viewed in Fig. 8.

As shown in upper area of Fig. 8(a), the first component pad 10 has the non-joined region 14 partially cut away to define the cutout 68. The region of the second component pad 20 corresponding to the cutout 68 is colored differently from the other regions to define a colored region 69. In this way, the colored region 69 can be visually recognized through the cutout 68, i.e., the cutout 6 8 and the colored region 69 cooperate with each other to define the indicator element.

The second component pad 20 is exposed as the first component pad 10 is peeled off from the composite pad 1 and this second component pad 20 has its non-joined region 24 partially cut away so as to define a cutout 70. The region of the third component pad 30 corresponding to the cutout 70 is colored differently from the other regions to define a colored region 71. With such an arrangement, the colored region 71 is visually recognized through the cutout 70, i.e., the colored region 71 and the cutout 70 cooperate with each other to form the indicator element. The region 71 may be colored differently from the colored region 69.

The composite pad 1 according to Example 6 is formed with the cutout regions 68, 70 through which the colored regions 69, 71 of the underlying component pad can be visually recognized. Such arrangement facilitates the non-joined regions 14, 24 to be recognized. It should be understood that the colored regions 69, 71 are not essential features and may be eliminated without departing from the scope of the invention. Even if none of these colored regions 69, 71 is provided, the cutouts 68, 70 effectively assist the wearer to recognize the non-joined regions 14, 24.

### EXAMPLE 7

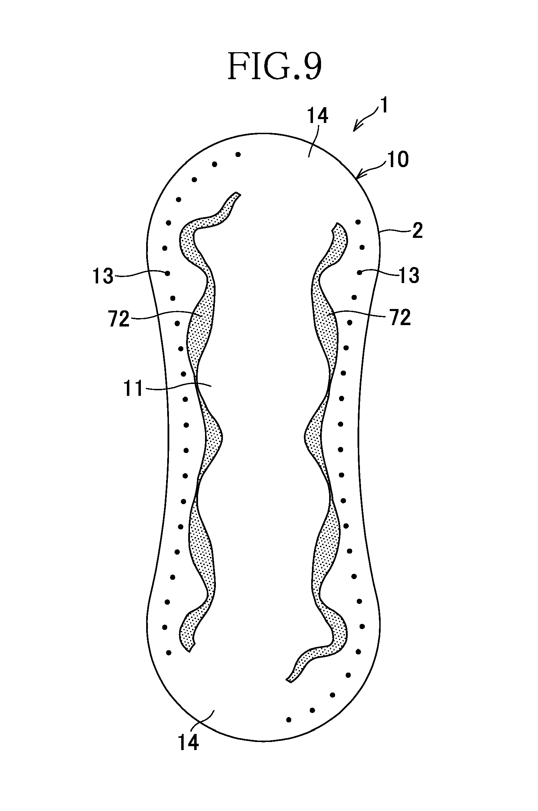

Fig. 9 is a view similar to Fig. 5, illustrating an indicator element 72 used by Example 7. The first component pad 10 is formed along transversely opposite side edges with two temporarily joined regions 13, respectively, and two non-joined regions 14 are defined between these two temporarily joined regions 13 as shown in upper and lower areas of Fig. 9. In this Example, the regions including none of the indicator elements can be recognized as the non-joined regions 14 merely by displaying the indicator elements 72 in the respective temporarily joined regions 13. Graphics of knot-less ribbon pieces are displayed as the indicator elements 72.

It should be appreciated that it is not essential for the indicator elements 72 to fall precisely on the associated temporarily joined regions 13 and the respective graphics 64 may be sufficiently nearby the associated temporarily joined regions 13 to distinguish the temporarily joined regions 13 from the non-joined regions 14. It is possible to use graphics of ivy leaves as the indicator elements 72, for example. These indicator elements 72 preferably include graphics which can be drawn so as to be contiguous one to another so that the regions in which the graphics are interrupted can be recognized as the non-joined regions 14.

In the respective Examples, the indicator element may be formed not only by printing or the like technique but also by locally embossing the surfaces of the respective component pads. When the printing or the like technique is employed, the indicator element may be printed directly on the liquid-absorbent sheet or on the liquid-impervious sheet so that the indicator element can be visually recognized through the liquid-absorbent sheet. It is also possible to use a letter, numeral or the like or a combination of such letters or the like, and a graphic, symbol, pattern or the like as the indicator element. While the respective Examples have been described on the assumption that each of them is the trilaminar composite pad, the invention is not limited to the trilaminar composite pad and the number of the component pads may be optional so far as the number of the component pads is two or more. However, it is important to temporarily join the respective component pads along the temporarily joined regions 13, 23, 33 without unintentionally being peeled off these component pads from the composite pad put on the wearer's body.

In the lowermost third component pad 30, fibrous non-woven fabric such as spun bond/melt blown/spun bond (SMS) non-woven fabric may be additionally layered on the lower surface of the third component pad 30 and the joining means 40 is provided on this fibrous non-woven fabric. The non-woven fabric additionally layered on the third component pad 30 serves as a reinforcing member preventing the third component pad 30 from being damaged in the course of peeling off the third component pad 30 from the wearer's garment such as briefs for the purpose of exchanging the composite pad 1. By using the fibrous non-woven fabric as the reinforcing member, stiffness of the liquid-impervious sheet 32 constituting the third component pad 30 can be reduced and thereby feeling of discomfort which might otherwise be experienced by the wearer during use of the composite pad 1 can be alleviated.

## Claims

1. A disposable bodily fluid absorbent composite pad having a longitudinal direction and a transverse direction and comprising a skin-facing side and a non-skin-facing side opposite to the skin-facing side and two or more component pads each including an upper liquid-absorbent sheet on the skin-facing side and a lower liquid-impervious sheet on the non-skin-facing side and layered one on another, wherein:
the two or more component pads respectively comprise temporarily joined regions along which the component pads are temporarily joined to each other so as to be peeled off from other component pads and non-joined regions; and
each of the component pads is formed with an indicator element adapted to be visually recognized through the liquid-absorbent sheet and the indicator elements are formed in the non-joined regions defined along peripheral edges of the component pads and adapted to indicate at least partially the non-joined regions.

2. The disposable bodily fluid absorbent composite pad defined by Claim 1, wherein the temporarily joined regions are formed substantially in continuous fashion along peripheral edges of the component pads and the non-joined regions are defined along partial segments of the peripheral edges so as to interrupt the substantially continuous temporarily joined regions.

3. The disposable bodily fluid absorbent composite pad defined by Claim 1 or 2, wherein the indicator element comprises at least any one of a letter, a symbol and a graphic displayed in the non-joined regions.

4. The disposable bodily fluid absorbent composite pad defined by any one of Claims 1 through 3, wherein the indicator element comprises at least any one of a letter, a symbol and a graphic serving to indicate the non-joined regions.

5. The disposable bodily fluid absorbent composite pad defined by any one of Claims 1 through 4, wherein the indicator element is defined by coloring of the non-joined regions.

6. The disposable bodily fluid absorbent composite pad defined by any one of Claims 1 through 5, wherein the indicator element comprises cutouts formed by partially cutting away the component pads.
